(19) Europäisches Patentamt
European Patent Office
Office européen des brevets

(11) **EP 4 321 209 B1**

(12) **EUROPEAN PATENT SPECIFICATION**

| | |
|---|---|
| (45) Date of publication and mention of the grant of the patent:<br>**22.04.2026 Bulletin 2026/17** | (51) International Patent Classification (IPC):<br>**_A61N 5/10_** (2006.01) |
| (21) Application number: **23159618.0** | (52) Cooperative Patent Classification (CPC):<br>**A61N 5/1077;** A61N 5/1031; A61N 2005/109 |
| (22) Date of filing: **02.03.2023** | |

(54) **DOSE CONTROL SYSTEM**

DOSISSTEUERSYSTEM

SYSTÈME DE CONTRÔLE DE DOSE

| | |
|---|---|
| (84) Designated Contracting States:<br>**AL AT BE BG CH CY CZ DE DK EE ES FI FR GB GR HR HU IE IS IT LI LT LU LV MC ME MK MT NL NO PL PT RO RS SE SI SK SM TR** | (72) Inventor: **LIN, Tzung-Yi**<br>**30261 Hsinchu County (TW)** |
| (30) Priority: **11.08.2022 TW 111130177** | (74) Representative: **Patentship**<br>**Patentanwaltsgesellschaft mbH**<br>**Paul-Gerhardt-Allee 50**<br>**81245 München (DE)** |
| (43) Date of publication of application:<br>**14.02.2024 Bulletin 2024/07** | (56) References cited:<br>**WO-A1-2022/002223 WO-A1-2022/002224**<br>**WO-A1-2022/002230 WO-A1-2022/111243**<br>**CN-A- 106 975 162 US-A1- 2018 326 225** |
| (73) Proprietor: **Heron Neutron Medical Corp.**<br>**Zhubei City, Hsinchu County 30261 (TW)** | |

**Description**

**BACKGROUND OF THE INVENTION**

**Field of the Invention**

The present disclosure relates in general to the field of radiation oncology, and it relates particularly to a dose control system.

**Description of the Related Art**

Currently, radiation oncology systems that are widely known, such as boron neutron capture therapy (BNCT) systems, estimate the dose rate (i.e., dose taken within a unit of time) and the irradiation time without considering the drug concentration in plasma of the patient, or under the presumption that the drug concentration in plasma of the patient is a specific theoretical value. However, since the dose rate may be affected by the drug concentration in plasma of the patient, and since the real drug concentration in plasma of the patient may not be equal to the presumed value, known systems may be prone to underestimate or overestimate dose delivered to the patient due to incorrect estimation of the dose rate.
In view of the issues described above, there is a need for a dose control system that calculates the dose rate and the irradiation time in order to control the irradiation device to irradiate the patient for the appropriate amount of time. Such exemplary system is described in publication WO 2022/002223 A1.

**BRIEF SUMMARY OF THE INVENTION**

A dose control system is provided by the present disclosure. The dose control system includes a processing device configured to execute the steps of (i) obtaining the real drug concentration in plasma of a patient; (ii) calculating a corrected three-dimensional dose rate distribution datum based on the real drug concentration in plasma and a set of correction coefficients; (iii) calculating irradiation time based on the corrected dose rate distribution datum and the prescribed dose distribution datum; and (iv) controlling an irradiation device to irradiate the patient for the irradiation time. The set of correction coefficients is generated by the dose control system based on multiple reference drug concentrations in plasma and multiple reference three-dimensional dose rate distribution data corresponding to the reference drug concentrations in plasma.
In an embodiment, the set of correction coefficients includes one of the reference drug concentrations in plasma, one of the reference dose rate distribution data, and the dose rate change with drug concentration in plasma distribution datum.
In an embodiment, the calculation of the corrected dose rate distribution datum uses Formula <I> as follows:

$$Dose\_rate(Drug_{real}, r) = Dose\_rate(Drug_{ref}, r) + \frac{\partial Dose\_rate(Drug, r)}{\partial Drug}\left(Drug_{real} - Drug_{ref}\right) \ .....<I>$$

In Formula <I>, the corrected dose rate distribution datum is represented by $Dose\_rate(Drug_{real}, r)$, the real drug concentration in plasma is represented by $Drug_{real}$, a spatial location is represented by r, the reference dose rate distribution datum is represented by $Dose\_rate(Drug_{ref}, r)$, the reference drug concentrations in plasma is represented by $Drug_{ref}$, and the dose rate change with drug concentration in plasma distribution datum is represented by $\frac{\partial Dose\_rate(Drug, r)}{\partial Drug}$.

In an embodiment, the processing device is further configured to obtain the real beam intensity of the irradiation device. The set of correction coefficients further includes a reference beam intensity corresponding to the reference drug concentration in plasma and the reference dose rate distribution datum.
In an embodiment, the calculation of the corrected dose rate distribution datum uses Formula <II> as follows:

$$Dose\_rate(Beam_{real}, Drug_{real}, r) = \frac{Beam_{real}}{Beam_{ref}}\left[Dose\_rate(Beam_{ref}, Drug_{ref}, r) + \right.$$

$$\left. \frac{\partial Dose\_rate(Beam_{ref}, Drug, r)}{\partial Drug}(Drug_{real} - Drug_{ref})\right] \ldots\ldots <II>$$

In Formula <II>, the corrected dose rate distribution datum is represented by $Dose\_rate(Beam_{real}, Drug_{real}, r)$, the real beam intensity is represented by $Beam_{real}$, the real drug concentration in plasma is represented by $Drug_{ref}$, a spatial location is represented by r, the reference dose rate distribution datum is represented by $Dose\_rate(Beam_{ref}, Drug_{ref}, r)$, the reference beam intensity is represented by $Beam_{ref}$, the reference drug concentration in plasma is represented by $Drug_{ref}$, and the dose rate change with drug concentration in plasma distribution datum is represented by

$$\frac{\partial Dose\_rate(Beam_{ref}, Drug, r)}{\partial Drug}.$$

In an embodiment, the real beam intensity is the neutron flux obtained using a neutron measuring device, or the proton current obtained using a beam measuring device configured by an accelerator.

In an embodiment, the processing device is further configured to create the time-dependent function of drug concentration in plasma based on multiple real drug concentrations in plasma, and to create the time-dependent function of beam intensity based on multiple real beam intensities. The processing device is further configured to calculate the time-dependent function of dose rate distribution based on the time-dependent function of drug concentration in plasma, the time-dependent function of beam intensity, and the set of correction coefficients. The processing device is further configured to calculate the time-dependent function of accumulated dose distribution based on the time-dependent function of dose rate distribution. The processing device is further configured to calculate the remaining irradiation time based on the prescribed dose rate distribution datum, the time-dependent function of accumulated dose distribution, and the time-dependent function of dose rate distribution. The processing device is further configured to control the irradiation device to irradiate the patient for the remaining irradiation time.

In an embodiment, the calculation of the time-dependent function of dose rate distribution uses Formula <III> as follows:

$$Dose\_rate(Beam(t), Drug(t), r) = \frac{Beam(t)}{Beam_{ref}}\left[Dose\_rate(Beam_{ref}, Drug_{ref}, r) + \right.$$

$$\left. \frac{\partial Dose\_rate(Beam_{ref}, Drug, r)}{\partial Drug}(Drug(t) - Drug_{ref})\right] \ldots\ldots <III>$$

In Formula <III>, the time-dependent function of dose rate distribution is represented by $Dose\_rate(Beam(t), Drug(t), r)$, the time-dependent function of beam intensity is represented by $Beam(t)$, the time-dependent function of drug concentration in plasma is represented by $Drug(t)$, a spatial location is represented by r, the reference dose rate distribution datum is represented by $Dose\_rate(Beam_{ref}, Drug_{ref}, r)$, the reference beam intensity is represented by $Beam_{ref}$, the reference drug concentration in plasma is represented by $Drug_{ref}$, the dose rate change with drug concentration in plasma distribution datum is represented by $\frac{\partial Dose\_rate(Beam_{ref}, Drug, r)}{\partial Drug}$. Correspondingly, the calculation of the time-dependent function of accumulated dose distribution uses Formula <IV> as follows:

$$Dose(t, r) = \int Dose\_rate(Beam(t), Drug(t), r)\,dt \ldots\ldots <IV>$$

In Formula <IV>, the time-dependent function of accumulated dose distribution is represented by $Dose(t, r)$, and the time-dependent function of dose rate distribution is represented by $Dose\_rate(Beam(t), Drug(t), r)$.

The dose control system provided by the present disclosure calculates the dose rate and the irradiation time so as to control the irradiation device to irradiate the patient for an appropriate amount of time. Accordingly, underestimating or overestimating delivered dose to the patient due to incorrect estimation of the dose rate can be avoided.

## BRIEF DESCRIPTION OF THE DRAWINGS

The present disclosure can be better understood by reading the subsequent detailed description and examples with references made to the accompanying drawings. Additionally, it should be appreciated that in the flow diagram of the present disclosure, the order of execution for each block can be changed, and/or some of the blocks can be changed, eliminated, or combined.

FIG. 1 is a schematic diagram illustrating the application scenario of the processing device of the dose control system, according to an embodiment of the present disclosure;

FIG. 2 is a flow diagram illustrating the dose control method executed by the processing device of the dose control system, according to an embodiment of the present disclosure; and

FIG. 3 is a flow diagram illustrating the dose control method executed by the processing device of the dose control system with the consideration of how the parameters change with time, according to an embodiment of the present disclosure.

## DETAILED DESCRIPTION OF THE INVENTION

The following description provides embodiments of the invention, which are intended to describe the invention, but is not intended to limit the invention. For the actual inventive content, reference must be made to the scope of the claims.

In each of the following embodiments, the same reference numbers represent identical or similar elements or components.

Ordinal terms used in the claims, such as "first," "second," "third," etc., are only for convenience of explanation, and do not imply any precedence relation between one another.

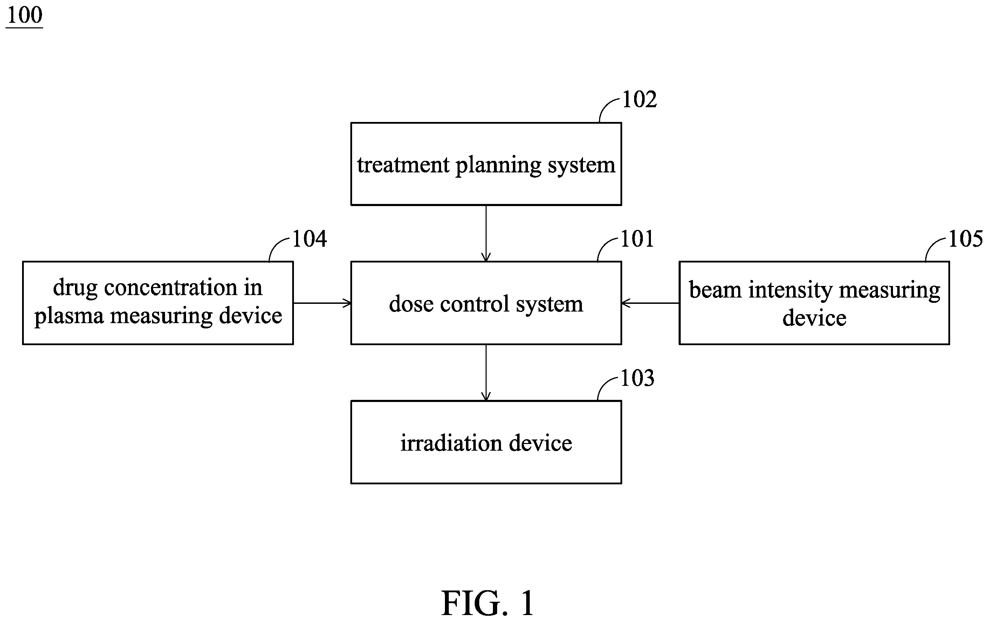

FIG. 1 is a schematic diagram illustrating the application scenario 100 of a dose control system 101, according to an embodiment of the present disclosure. As shown in FIG. 1, in the application scenario 100, the dose control system 101 may receive data provided by the treatment planning system 102, the drug concentration in plasma measuring device 104, and the beam intensity measuring device 105, and calculate the irradiation time, in order to control the irradiation device 103 to irradiate the patient for the irradiation time. The dose control system 101 may pass through wired or wireless connection to each of the treatment planning system 102, the drug concentration in plasma measuring device 104, and the beam intensity measuring device 105, to receive data provided by each of the treatment planning system 102, the drug concentration in plasma measuring device 104, and the beam intensity measuring device 105. Alternatively, the data provided by the treatment planning system 102, the drug concentration in plasma measuring device 104, and the beam intensity measuring device 105 may be manually input to the dose control system by human. The dose control system 101 may pass through wired or wireless connection to the irradiation device 103, and control the irradiation device 103 to irradiate for the time required through commands or instructions. Alternatively, the irradiation time calculated by the dose control system 101 may be displayed on a displaying device (e.g., LCD display, LED display, OLED display, or plasma display), allowing a human to manipulate the irradiation device 103 manually, so as to control the irradiation device 103 to irradiate the patient for the irradiation time.

The dose control system may be a computer system, such as a personal computer (e.g., laptop computer or notebook computer) or a server computer running an operating system (e.g., Windows, Mac OS, Linux, UNIX..., etc.). The dose control system 101 includes a processing device. The processing device may be any device used for executing instructions, such as a central processing unit (CPU), a microprocessor, a controller, a microcontroller, or a state machine. The dose control system 101 may further include a storage device. The storage device may be a storage device including electrically-erasable programmable read-only memory (EEPROM), flash memory, or non-volatile random access memory (NVRAM), such as hard disk drives (HDD), solid state drives (SSD), or optical disks.

The treatment planning system 102 may be a computer system, such as a personal computer (e.g., laptop computer or notebook computer) or a server computer running an operating system (e.g., Windows, Mac OS, Linux, UNIX..., etc.). The treatment planning system 102 includes a processing device. The processing device may be any device used for executing instructions, such as a central processing unit (CPU), a microprocessor, a controller, a microcontroller, or a state machine.

Although the dose control system 101 and the treatment planning system 102 are drawn as different systems, the present disclosure is not limited thereto. In some embodiments of the present disclosure, the dose control system and the treatment planning system 102 may be different modules or units of the same computer system.

In an embodiment of the present disclosure, the treatment planning system 102 is used for providing the three-dimensional (3D) dose rate distribution data (i.e., dose rates of each point in the 3D space) corresponding to multiple drug concentrations in plasma, such as the dose rate distribution data when the drug concentration in plasma is 20 ppm, 25 ppm, 30 ppm..., etc. These dose rate distribution data may be measured through clinical experiments, or may be calculated using specific algorithms. In an embodiment, the dose rate distribution data are calculated based on organ tissue distribution data, drug absorptance distribution data, material composition parameter data, volume homogenization size, particle tally size, beam parameters, and drug concentration in plasma, with the use of volume homogenization algorithm and Monte Carlo particle transport algorithm. In some embodiments, the treatment planning system 102 further converts the dose rate distribution data into a set of correction coefficients, allowing the dose control system 101 to calculate the corrected dose rate distribution and the irradiation time. In other embodiments, the treatment planning system 102 provides the dose rate distribution data corresponding to multiple drug concentrations in plasma to the dose control system 101, so that the dose control system 101 converts the dose rate distribution data into the set of correction coefficients, allowing to calculate the corrected dose rate distribution and irradiation time subsequently.

The irradiation device 103 may be any neutron emitter for emitting neutrons to the tumor of the patient. The neutrons emitted will be absorbed by the drug (e.g., Boron-10) accumulated in the tumor of the patient, and then the particles with high energy (e.g., lithium-7 or $\alpha$ particles) will be generated to damage the cancer cells locally.

The drug concentration in plasma measuring device 104 may be an inductively coupled plasma mass spectrometry (ICP-MS), an inductively coupled plasma atomic emission spectroscopy (ICP-AES), or any device that may be used for measuring the concentration of drug (e.g., Boron-10) in plasma of the patient.

The beam intensity measuring device 105 may be a neutron measuring device or a beam measuring device configured by an accelerator. The beam intensity measuring device 105 is used for measuring the beam intensity of the irradiation device 103. If the beam intensity measuring device 105 is a neutron measuring device, the measured beam intensity will be neutron flux. If the beam intensity measuring device 105 is a beam measuring device configured by an accelerator, the measured beam intensity will be proton current. In an embodiment, the neutron measuring device and the beam measuring device configured by an accelerator can be a backup beam intensity measuring device 105 for one another, so as to avoid underestimating or overestimating delivered dose caused by the incorrect measurement of one of them.

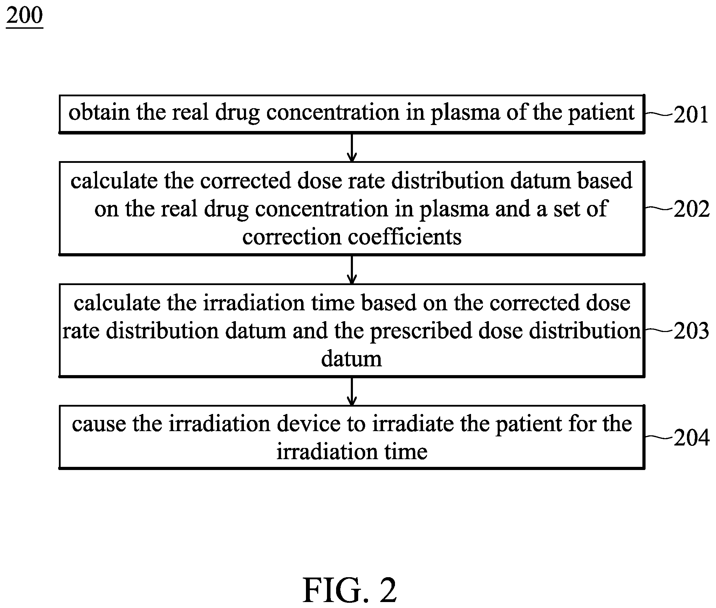

FIG. 2 is a flow diagram illustrating a dose control method 200 executed by the processing device of the dose control system 101 in FIG. 1, according to an embodiment of the present disclosure. The method does not form part of the invention. As shown in FIG. 2, method 200 includes steps 201-204.

Method 200 starts from step 201. In step 201, the real drug concentration in plasma of the patient is obtained. Then, method 200 proceeds to step 202.

In step 202, the corrected dose rate distribution datum is calculated based on the real drug concentration in plasma and a set of correction coefficients. Then, method 200 proceeds to step 203.

In step 203, the irradiation time is calculated based on the corrected dose rate distribution datum and the prescribed dose distribution datum. Then, method 200 proceeds to step 204.

In step 204, the irradiation device is controlled to irradiate the patient for the irradiation time.

In embodiments of the present disclosure, the real drug concentration in plasma can actually be measured using the measuring device (e.g., the drug concentration measuring device 104 in FIG. 1) at the moment just before the patient taking the radiation treatment, or at any time point during the whole process of irradiation.

In an embodiment of the present disclosure, since typically the real drug concentration in plasma of the patient may not be completely equal to the multiple drug concentrations in plasma provided by the treatment planning system 102, the drug concentrations in plasma and the corresponding multiple dose rate distribution data provided by the treatment planning system 102 are only for reference. Therefore, the dose rate distribution datum corresponding to the real drug concentration in plasma of the patient is calculated based on the set of correction coefficients, instead of directly applying one of the dose rate distribution data provided by the treatment planning system 102. The set of correction coefficients is generated based on multiple reference drug concentrations in plasma and the corresponding multiple reference dose rate distribution data provided by the treatment planning system 102. In some embodiments, the set of correction coefficients are converted from multiple reference drug concentrations in plasma and the corresponding multiple reference dose rate distribution data by the treatment planning system 102.

In an embodiment, the set of correction coefficients may include one of the reference drug concentrations in plasma and the corresponding reference dose rate distribution datum, and the dose rate change with drug concentration in plasma distribution (i.e., the dose rate change corresponding to each unit of change of drug concentration in plasma at each point in the 3D space) datum.

Accordingly, the calculation of the corrected dose rate distribution datum may use Formula <I> as follows:

$$Dose\_rate(Drug_{real}, r) = Dose\_rate(Drug_{ref}, r) + \frac{\partial Dose\_rate(Drug, r)}{\partial Drug}\left(Drug_{real} - \right.$$

$$\left. Drug_{ref}\right) \ldots <I>$$

In Formula <I>, the corrected dose rate distribution datum is represented by $Dose\_rate(Drug_{real}, r)$, the real drug concentration in plasma is represented by $Drug_{real}$, a spatial location is represented by $r$ ($r$ may represent a coordinate in the 3D spatial coordinate system), the reference dose rate distribution datum is represented by $Dose\_rate(Drug_{ref}, r)$,

the reference drug concentrations in plasma is represented by $Drug_{ref}$, and the dose rate change with drug concentration in plasma distribution datum is represented by $\frac{\partial Dose\_rate(Drug,r)}{\partial Drug}$ .

Corresponding to Formula <I>, the calculation of irradiation time in step 204 may use Formula <V> as follows:

$$T_f = \min\left(\frac{Prescription\_dose(r)}{Dose\_rate(Drug_{real},r)}\right) \ \ldots\ldots <\text{V}>$$

In formula <V>, the irradiation time is represented by $T_f$, the corrected dose rate distribution datum calculated using Formula <I> is represented by $Dose\_rate(Drug_{real}, r)$, the prescribed dose distribution (i.e., the prescribed dose at each points in the 3D space) datum is represented by $Prescription\_dose(r)$, and min() represents the minimal one of $\frac{Prescription\_dose(r)}{Dose\_rate(Drug_{real},r)}$ corresponding to all the r values (i.e., all locations). As such, overdosing may be avoided for any location of the patient.

In another embodiment, in view of that the dose rate may also be affected by the beam intensity, the set of correction coefficients may further include a reference beam intensity corresponding to the reference drug concentration in plasma and the reference dose rate distribution datum, besides one of reference drug concentrations in plasma, the reference dose rate distribution datum corresponding to the one of reference drug concentrations in plasma, and the dose rate change with drug concentration in plasma distribution datum that are previously described.

Accordingly, the calculation of the corrected dose rate distribution datum may use Formula <II> as follows:

$$Dose\_rate(Beam_{real}, Drug_{real}, r) = \frac{Beam_{real}}{Beam_{ref}}\left[Dose\_rate\big(Beam_{ref}, Drug_{ref}, r\big) + \right.$$

$$\left. \frac{\partial Dose\_rate\big(Beam_{ref},Drug,r\big)}{\partial Drug}\big(Drug_{real} - Drug_{ref}\big)\right] \ \ldots\ldots <\text{II}>$$

In Formula <II>, the corrected dose rate distribution datum is represented by $Dose\_rate(Beam_{real}, Drug_{real}, r)$, the real beam intensity is represented by $Beam_{real}$, the real drug concentration in plasma is represented by $Drug_{ref}$, a spatial location is represented by $r$, the reference dose rate distribution datum is represented by $Dose\_rate(Beam_{ref}, Drug_{ref}, r)$, the reference beam intensity is represented by $Beam_{ref}$, the reference drug concentration in plasma is represented by $Drug_{ref}$, and the dose rate change with drug concentration in plasma distribution datum is represented by $\frac{\partial Dose\_rate\big(Beam_{ref},Drug,r\big)}{\partial Drug}$ .

Corresponding to Formula <II>, the calculation of the irradiation time in step 204 may use Formula <VI> as follows:

$$T_f = \min\left(\frac{Prescription\_dose(r)}{Dose\_rate(Beam_{real},Drug_{real},r)}\right) \ \ldots\ldots <\text{VI}>$$

In Formula <VI>, the irradiation time is represented by $T_f$, the corrected dose rate distribution datum calculated using Formula <II> is represented by $Dose\_rate(Beam_{real}, Drug_{real}, r)$, the prescribed dose distribution (i.e., the prescribed dose at each points in the 3D space) datum is represented by $Prescription\_dose(r)$, and min() represents the minimal one of $\frac{Prescription\_dose(r)}{Dose\_rate(Beam_{real},Drug_{real},r)}$ corresponding to all the $r$ values (i.e., all locations). As such, overdosing may be avoided for any location of the patient.

In some embodiments, it is considered that the drug concentration in plasma of the patient typically decreases or increases with time gradually during the irradiation, such that the drug concentration in plasma that is measured at the beginning (e.g., at the moment just before the patient taking the radiation treatment) may not be equal to the real drug concentration in plasma during the irradiation, and the dose rate will not be constant anymore. Therefore, there is a need to consider the real drug concentration in plasma change with time, and to calculate the time-dependent function of dose rate distribution (i.e., the correspondence between time and dose rate distribution), in order to estimate the remaining irradiation time more accurately and immediately.

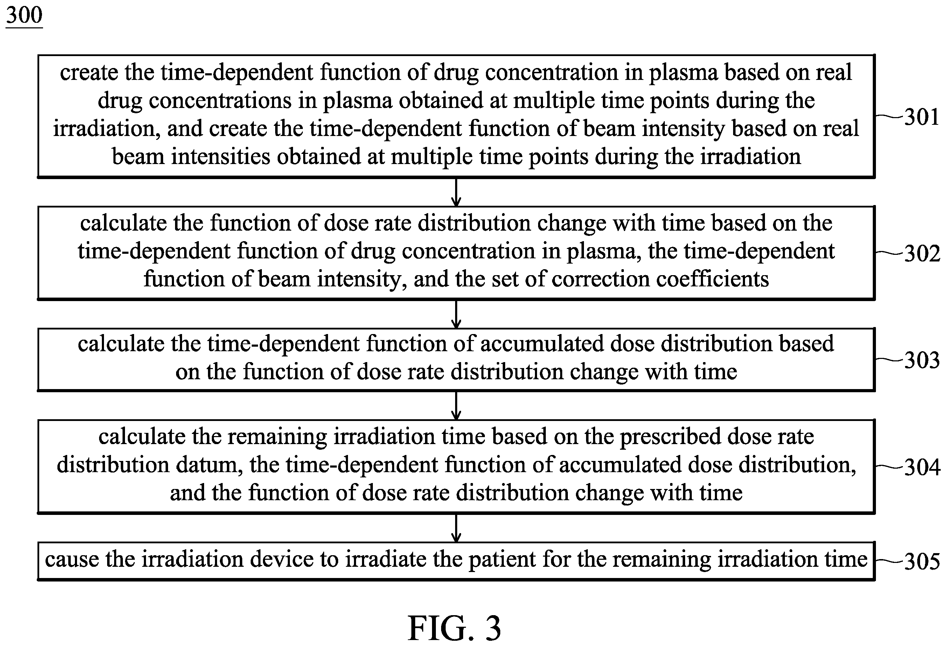

FIG. 3 is a flow diagram illustrating a dose control method 300 executed by the processing device of the dose control system in FIG. 1. The method does not form part of the invention. As shown in FIG. 3, method 300 includes steps 301-305.

Method 300 starts from step 301. In step 301, the time-dependent function of drug concentration in plasma (i.e., the correspondence between time and drug concentration in plasma) is created based on real drug concentrations in plasma obtained at multiple time points during the irradiation, and the time-dependent function of beam intensity (i.e., the correspondence between time and beam intensity) is created based on real beam intensities obtained at multiple time points during the irradiation. Then, method 300 proceeds to step 302.

In step 302, the time-dependent function of dose rate distribution (i.e., the correspondence between time and dose rate distribution) is calculated based on the time-dependent function of drug concentration in plasma, the time-dependent function of beam intensity, and the set of correction coefficients. Then, method 300 proceeds to step 303.

In step 303, the time-dependent function of accumulated dose distribution (i.e., the correspondence between time and the accumulated doses at each point in the 3D space) is calculated based on the time-dependent function of dose rate distribution. Then, method 300 proceeds to step 304.

In step 304, the remaining irradiation time is calculated based on the prescribed dose rate distribution datum, the time-dependent function of accumulated dose distribution, and the time-dependent function of dose rate distribution. Then, method 300 proceeds to step 305.

In step 305, the irradiation device is controlled to irradiate the patient for the remaining irradiation time.

In an embodiment, the calculation of the time-dependent function of dose rate distribution may use Formula <III> as follows:

$$Dose\_rate(Beam(t), Drug(t), r) = \frac{Beam(t)}{Beam_{ref}} \left[ Dose\_rate(Beam_{ref}, Drug_{ref}, r) + \right.$$

$$\left. \frac{\partial Dose\_rate(Beam_{ref}, Drug, r)}{\partial Drug} (Drug(t) - Drug_{ref}) \right] \dots \dots <III>$$

In Formula <III>, the time-dependent function of dose rate distribution is represented by $Dose\_rate(Beam(t), Drug(t), r)$, the time-dependent function of beam intensity is represented by $Beam(t)$, the time-dependent function of drug concentration in plasma is represented by $Drug(t)$, a spatial location is represented by $r$, the reference dose rate distribution datum is represented by $Dose\_rate(Beam_{ref}, Drug_{ref}, r)$, the reference beam intensity is represented by $Beam_{ref}$, the reference drug concentration in plasma is represented by $Drug_{ref}$, the dose rate change with drug concentration in plasma distribution datum is represented by $\frac{\partial Dose\_rate(Beam_{ref}, Drug, r)}{\partial Drug}$, and t may represent any time point during the irradiation (e.g., the $t^{th}$ second from the beginning of irradiation).

Corresponding to Formula <III>, the calculation of the time-dependent function of accumulated dose distribution may use Formula <IV> as follows:

$$Dose(t, r) = \int Dose\_rate(Beam(t), Drug(t), r) \, dt \dots \dots <IV>$$

In Formula <IV>, the time-dependent function of accumulated dose distribution is represented by $Dose(t, r)$, and the time-dependent function of dose rate distribution calculated by Formula <III> is represented by $Dose\_rate(Beam(t), Drug(t), r)$.

Corresponding to Formula <III> and Formula <IV>, the calculation of the remaining irradiation time may use Formula <VII> as follows:

$$T_{remain} = \min \left( \frac{Prescription\_dose(r) - Dose(t, r)}{Dose\_rate(Beam(t), Drug(t), r)} \right) \dots \dots <VII>$$

In Formula <VII>, the remaining irradiation time is represented by $T_{remain}$, the time-dependent function of dose rate distribution calculated by Formula <III> is represented by $Dose\_rate(Beam(t), Drug(t), r)$, the prescribed dose distribution (i.e., the prescribed dose at each points in the 3D space) is represented by $Prescription\_dose(r)$, and min() represents the minimal one of $\frac{Prescription\_dose(r) - Dose(t, r)}{Dose\_rate(Beam(t), Drug(t), r)}$ corresponding to all the $r$ values (i.e., all locations). As such, overdosing may be avoided for any location of the patient.

In further embodiments, the expected total dose $Dose(r)$ for the whole course of treatment may be calculated based on the time-dependent function of accumulated dose distribution and the remaining irradiation time $T_{remain}$. Specifically, by adding the current time point and $T_{remain}$, the expected total irradiation time $T_{total}$ for the whole course of treatment may be obtained (e.g., if currently the patient has been irradiated for 20 minutes, and the remaining irradiation time is 10 minutes, then the total irradiation time will be 20 + 10 = 30 minutes). Afterwards, $T_{total}$ is substituted into Formula <IV>, in other words $\int_0^{T_{total}} Dose\_rate(Beam(t), Drug(t), r)\, dt$ is calculated, and the expected total dose $Dose(r)$ for the whole course of treatment may be obtained.

The methods described above can be carried out using computer-executable instructions. For example, these instructions may include instructions and data that allow a general-purpose computer, a special-purpose computer, or a special-purpose processing device to execute specific functions or a set of functions. Some of the computer resources may be accessed through a network.

The dose control system provided by the present disclosure calculates the dose rate and the irradiation time so as to control the irradiation device to irradiate the patient for an appropriate amount of time. Accordingly, underestimating or overestimating delivered dose to the patient due to incorrect estimation of the dose rate can be avoided.

The above paragraphs are described with multiple aspects. Obviously, the teachings of the specification may be performed in multiple ways. Any specific structure or function disclosed in the examples is only a representative situation. According to the teachings of the specification, it should be noted by those skilled in the art that any aspect disclosed may be performed individually, or that more than two aspects could be combined and performed.

While the invention has been described by way of example and in terms of the preferred embodiments, it should be understood that the invention is not limited to the disclosed embodiments. The invention is defined by the appended claims.

**Claims**

1.   A dose control system (101), comprising a processing device configured to execute the following steps:

   obtaining a real drug concentration in plasma of a patient;
   calculating a corrected three-dimensional dose rate distribution datum based on the real drug concentration in plasma and a set of correction coefficients;
   calculating irradiation time based on the corrected dose rate distribution datum and a prescribed dose distribution datum; and
   controlling an irradiation device (103) to irradiate the patient for the irradiation time;
   wherein the set of correction coefficients is generated by the dose control system based on multiple reference drug concentrations in plasma and multiple reference three-dimensional dose rate distribution data corresponding to the reference drug concentrations in plasma.

2.   The dose control system (101) as claimed in claim 1, wherein the set of correction coefficients comprises one of the reference drug concentrations in plasma, one of the reference dose rate distribution data, and a dose rate change with drug concentration in plasma distribution datum.

3.   The dose control system (101) as claimed in claim 2, wherein the calculation of the corrected dose rate distribution datum uses the following formula:

$$Dose\_rate(Drug_{real}, r)$$
$$= Dose\_rate(Drug_{ref}, r)$$
$$+ \frac{\partial Dose\_rate(Drug, r)}{\partial Drug}(Drug_{real} - Drug_{ref})$$

wherein the corrected dose rate distribution datum is represented by $Dose\_rate(Drug_{real}, r)$, the real drug concentration in plasma is represented by $Drug_{real}$, a spatial location is represented by r, the reference dose rate distribution datum is represented by $Dose\_rate(Drug_{ref}, r)$, the reference drug concentrations in plasma is represented by $Drug_{ref}$, and the dose rate change with drug concentration in plasma distribution datum is represented by $\frac{\partial Dose\_rate(Drug, r)}{\partial Drug}$.

4. The dose control system (101) as claimed in claim 2, wherein the processing device is further configured to obtain a real beam intensity of the irradiation device (103);
wherein the set of correction coefficients further comprises a reference beam intensity corresponding to the reference drug concentration in plasma and the reference dose rate distribution datum.

5. The dose control system (101) as claimed in claim 4, wherein the calculation of the corrected dose rate distribution datum uses the following formula:

$$Dose\_rate(Beam_{real}, Drug_{real}, r)$$

$$= \frac{Beam_{real}}{Beam_{ref}} \left[ Dose\_rate(Beam_{ref}, Drug_{ref}, r) \right.$$

$$\left. + \frac{\partial Dose\_rate(Beam_{ref}, Drug, r)}{\partial Drug} (Drug_{real} - Drug_{ref}) \right]$$

wherein the corrected dose rate distribution datum is represented by $Dose\_rate(Beam_{real}, Drug_{real}, r)$, the real beam intensity is represented by $Beam_{real}$, the real drug concentration in plasma is represented by $Drug_{ref}$, a spatial location is represented by r, the reference dose rate distribution datum is represented by $Dose\_rate(Beam_{ref}, Drug_{ref}, r)$, the reference beam intensity is represented by $Beam_{ref}$, the reference drug concentration in plasma is represented by $Drug_{ref}$, and the dose rate change with drug concentration in plasma distribution datum is represented by $\frac{\partial Dose\_rate(Beam_{ref}, Drug, r)}{\partial Drug}$.

6. The dose control system (101) as claimed in claim 4, wherein the real beam intensity is a neutron flux obtained using a neutron measuring device, or a proton current obtained using a beam measuring device (105) configured by an accelerator.

7. The dose control system (101) as claimed in claim 2, wherein the processing device is further configured to execute the following steps:

creating a time-dependent function of drug concentration in plasma based on multiple real drug concentrations in plasma, and creating a time-dependent function of beam intensity based on multiple real beam intensities, wherein the real drug concentrations in plasma and the real beam intensities are obtained at multiple time points during irradiation;
calculating a time-dependent function of dose rate distribution based on the time-dependent function of drug concentration in plasma, the time-dependent function of beam intensity, and the set of correction coefficients;
calculating a time-dependent function of accumulated dose distribution based on the time-dependent function of dose rate distribution;
calculating remaining irradiation time based on the prescribed dose rate distribution datum, the time-dependent function of accumulated dose distribution, and the time-dependent function of dose rate distribution; and
controlling the irradiation device (103) to irradiate the patient for the remaining irradiation time.

8. The dose control system (101) as claimed in claim 7, wherein the calculation of the time-dependent function of dose rate distribution uses the following formula:

$$Dose\_rate(Beam(t), Drug(t), r)$$

$$= \frac{Beam(t)}{Beam_{ref}}\left[Dose\_rate\big(Beam_{ref}, Drug_{ref}, r\big)\right.$$

$$\left. + \frac{\partial Dose\_rate\big(Beam_{ref}, Drug, r\big)}{\partial Drug}\big(Drug(t) - Drug_{ref}\big)\right]$$

wherein the time-dependent function of dose rate distribution is represented by $Dose\_rate(Beam(t), Drug(t), r)$, the time-dependent function of beam intensity is represented by $Beam(t)$, the time-dependent function of drug concentration in plasma is represented by $Drug(t)$, a spatial location is represented by $r$, the reference dose rate distribution datum is represented by $Dose\_rate(Beam_{ref}, Drug_{ref}, r)$, the reference beam intensity is represented by $Beam_{ref}$, the reference drug concentration in plasma is represented by $Drug_{ref}$, the dose rate change with drug concentration in plasma distribution datum is represented by $\frac{\partial Dose\_rate(Beam_{ref}, Drug, r)}{\partial Drug}$; and

wherein the calculation of the time-dependent function of accumulated dose distribution uses the following formula:

$$Dose(t, r) = \int Dose\_rate(Beam(t), Drug(t), r)\, dt$$

wherein the time-dependent function of accumulated dose distribution is represented by $Dose(t, r)$, and the time-dependent function of dose rate distribution is represented by $Dose\_rate(Beam(t), Drug(t), r)$.

**Patentansprüche**

1. Ein Dosissteuersystem (101), umfassend eine Verarbeitungsvorrichtung, die zur Ausführung der folgenden Schritte ausgebildet ist:

   Ermitteln einer tatsächlichen Arzneimittelkonzentration im Plasma eines Patienten;
   Berechnen eines korrigierten dreidimensionalen Dosisleistungsverteilungsdatensatzes auf der Grundlage der tatsächlichen Arzneimittelkonzentration im Plasma und eines Satzes von Korrekturkoeffizienten;
   Berechnen der Bestrahlungszeit auf Grundlage des korrigierten Dosisleistungsverteilungsdatensatzes und eines vorgegebenen Dosisverteilungsdatensatzes; und
   Steuern einer Bestrahlungsvorrichtung (103) zum Bestrahlen des Patienten für die Bestrahlungszeit;
   wobei der Satz von Korrekturkoeffizienten von dem Dosissteuersystem auf der Grundlage mehrerer Referenzarzneimittelkonzentrationen im Plasma und mehrerer dreidimensionaler Referenz-Dosisleistungsverteilungsdaten, die den Referenzarzneimittelkonzentrationen im Plasma entsprechen, generiert wird.

2. Das Dosissteuersystem (101) nach Anspruch 1, wobei der Satz von Korrekturkoeffizienten eine der Referenzarzneimittelkonzentrationen im Plasma, eine der Referenz-Dosisleistungsverteilungsdaten, und einen Datensatz einer Dosisleistungsänderung mit der Verteilung der Arzneimittelkonzentration im Plasma umfasst.

3. Das Dosissteuersystem (101) nach Anspruch 2, wobei die Berechnung des korrigierten Dosisleistungsverteilungsdatensatzes die folgende Formel verwendet:

$$Dose\_rate(Drug_{real}, r)$$

$$= Dose\_rate\big(Drug_{ref}, r\big)$$

$$+ \frac{\partial Dose\_rate(Drug, r)}{\partial Drug}\big(Drug_{real} - Drug_{ref}\big)$$

wobei der korrigierte Dosisleistungsverteilungsdatensatz durch *Dose_rate*($Drug_{real}$, *r*) dargestellt wird, die tatsächliche Arzneimittelkonzentration im Plasma durch $Drug_{real}$ dargestellt wird, ein räumlicher Ort durch *r* dargestellt wird, der Referenz-Dosisleistungsverteilungsdatensatz durch *Dose_rate*($Drug_{ref}$, *r*) dargestellt wird, die Referenzarzneimittelkonzentrationen im Plasma durch $Drug_{ref}$ dargestellt wird, und der Datensatz der Dosisleistungsänderung mit der Verteilung der Arzneimittelkonzentration im Plasma durch $\frac{\partial Dose\_rate(Drug,r)}{\partial Drug}$ dargestellt wird.

4. Das Dosissteuersystem (101) nach Anspruch 2, wobei die Verarbeitungsvorrichtung ferner ausgebildet ist, um eine reale Strahlintensität der Bestrahlungsvorrichtung (103) zu erhalten;
wobei der Satz von Korrekturkoeffizienten ferner eine Referenzstrahlintensität umfasst, die der Referenzarzneimittelkonzentration im Plasma und dem Referenz-Dosisleistungsverteilungsdatensatz entspricht.

5. Das Dosissteuersystem (101) nach Anspruch 4, wobei die Berechnung des korrigierten Dosisleistungsverteilungsdatensatzes die folgende Formel verwendet:

$$Dose\_rate(Beam_{real}, Drug_{real}, r)$$

$$= \frac{Beam_{real}}{Beam_{ref}}\left[ Dose\_rate\big(Beam_{ref}, Drug_{ref}, r\big)\right.$$

$$\left. + \frac{\partial Dose\_rate\big(Beam_{ref}, Drug, r\big)}{\partial Drug}\big(Drug_{real} - Drug_{ref}\big)\right]$$

wobei der korrigierte Dosisleistungsverteilungsdatensatz durch *Dose_rate*($Beam_{real}$, $Drug_{real}$, *r*) dargestellt wird, die reale Strahlintensität durch $Beam_{real}$ dargestellt wird, die reale Arzneimittelkonzentration im Plasma durch $Drug_{ref}$ dargestellt wird, ein räumlicher Ort durch r dargestellt wird, der Referenz-Dosisleistungsverteilungsdatensatz durch *Dose_rate*($Beam_{ref}$, $Drug_{ref}$, *r*) dargestellt wird, die Referenzstrahlintensität durch $Beam_{ref}$ dargestellt wird, die Referenzarzneimittelkonzentration im Plasma durch $Drug_{ref}$ dargestellt wird, und der Datensatz der Dosisleistungsänderung mit der Verteilung der Arzneimittelkonzentration im Plasma durch $\frac{\partial Dose\_rate(Beam_{ref}, Drug, r)}{\partial Drug}$ dargestellt wird.

6. Das Dosissteuersystem (101) nach Anspruch 4, wobei die tatsächliche Strahlintensität ein Neutronenfluss ist, der mit einer Neutronenmessvorrichtung ermittelt wird, oder ein Protonenstrom, der mit einer Strahlmessvorrichtung (105) ermittelt wird, die durch einen Beschleuniger konfiguriert ist.

7. Das Dosissteuersystem (101) nach Anspruch 2, wobei die Verarbeitungsvorrichtung ferner ausgebildet ist, die folgenden Schritte auszuführen:

Erstellen einer zeitabhängigen Funktion der Arzneimittelkonzentration im Plasma auf der Grundlage mehrerer realer Arzneimittelkonzentrationen im Plasma, und Erstellen einer zeitabhängigen Funktion der Strahlintensität auf der Grundlage mehrerer realer Strahlintensitäten, wobei die realen Arzneimittelkonzentrationen im Plasma und die realen Strahlintensitäten zu mehreren Zeitpunkten während der Bestrahlung ermittelt werden;
Berechnen einer zeitabhängigen Funktion der Dosisleistungsverteilung auf der Grundlage der zeitabhängigen Funktion der Arzneimittelkonzentration im Plasma, der zeitabhängigen Funktion der Strahlintensität und des Satzes von Korrekturkoeffizienten;
Berechnen einer zeitabhängigen Funktion der akkumulierten Dosisverteilung auf der Grundlage der zeitabhängigen Funktion der Dosisleistungsverteilung;
Berechnen der verbleibenden Bestrahlungszeit auf Grundlage des vorgegebenen Dosisleistungsverteilungsdatensatzes, der zeitabhängigen Funktion der akkumulierten Dosisverteilung und der zeitabhängigen Funktion der Dosisleistungsverteilung; und
Steuern der Bestrahlungsvorrichtung (103) zur Bestrahlung des Patienten für die verbleibende Bestrahlungszeit.

8. Das Dosissteuersystem (101) nach Anspruch 7, wobei die Berechnung der zeitabhängigen Funktion der Dosisleistungsverteilung die folgende Formel verwendet:

$$Dose\_rate(Beam(t), Drug(t), r)$$

$$= \frac{Beam(t)}{Beam_{ref}} \left[ Dose\_rate\big(Beam_{ref}, Drug_{ref}, r\big) \right.$$

$$\left. + \frac{\partial Dose\_rate\big(Beam_{ref}, Drug, r\big)}{\partial Drug} \big(Drug(t) - Drug_{ref}\big) \right]$$

wobei die zeitabhängige Funktion der Dosisleistungsverteilung durch *Dose_rate(Beam*(*t*), *Drug*(*t*), *r*) dargestellt wird, die zeitabhängige Funktion der Strahlintensität durch *Beam*(*t*) dargestellt wird, die zeitabhängige Funktion der Arzneimittelkonzentration im Plasma durch *Drug*(*t*) dargestellt wird, ein räumlicher Ort durch r dargestellt wird, der Referenz-Dosisleistungsverteilungsdatensatz durch *Dose_rate*(*Beam_{ref}*, *Drug_{ref}*, *r*) dargestellt wird, die Referenzstrahlintensität durch *Beam_{ref}* dargestellt wird, die Referenzarzneimittelkonzentration im Plasma durch *Drug_{ref}* dargestellt wird, und der Datensatz der Dosisleistungsänderung mit der Verteilung der Arzneimittelkon-

zentration im Plasma durch $\dfrac{\partial Dose\_rate\big(Beam_{ref}, Drug, r\big)}{\partial Drug}$ dargestellt wird; und

wobei die Berechnung der zeitabhängigen Funktion der akkumulierten Dosisverteilung die folgende Formel verwendet:

$$Dose(t, r) = \int Dose\_rate(Beam(t), Drug(t), r)\, dt$$

wobei die zeitabhängige Funktion der akkumulierten Dosisverteilung durch *Dose*(*t, r*) dargestellt wird, und die zeitabhängige Funktion der Dosisleistungsverteilung durch *Dose_rate*(*Beam*(*t*), *Drug*(*t*), *r*) dargestellt wird.

## Revendications

1. Un système de contrôle de dose (101), comprenant un dispositif de traitement configuré pour exécuter les étapes suivantes :

   obtenir une concentration réelle du médicament dans le plasma d'un patient ;
   calcul d'une donnée de distribution de débit de dose tridimensionnelle corrigée basée sur la concentration réelle du médicament dans le plasma et un ensemble de coefficients de correction ;
   calcul du temps d'irradiation basé sur la distribution de dose corrigée et une distribution de dose prescrite ; et
   commander un dispositif d'irradiation (103) pour irradier le patient pendant la durée d'irradiation ;
   dans lequel l'ensemble des coefficients de correction est généré par le système de contrôle de dose sur la base de plusieurs concentrations de médicament de référence s dans le plasma et de plusieurs données de distribution de débit de dose tridimensionnelles de référence correspondant à la concentration de médicament de référence s dans le plasma.

2. Le système de contrôle de dose (101) selon la revendication 1, dans lequel l'ensemble des coefficients de correction comprend l'une des concentrations de médicament de référence s dans le plasma, l'une des données de distribution du débit de dose de référence et une donnée de distribution de changement de débit de dose avec la concentration de médicament dans le plasma.

3. Le système de contrôle de dose (101) selon la revendication 2, dans lequel le calcul de la donnée de distribution du débit de dose corrigée utilise la formule suivante :

$$Dose\_rate(Drug_{real}, r)$$
$$= Dose\_rate(Drug_{ref}, r)$$
$$+ \frac{\partial Dose\_rate(Drug, r)}{\partial Drug}(Drug_{real} - Drug_{ref})$$

dans lequel la donnée de distribution du débit de dose corrigée est représentée par $Dose\_rate(Drug_{real}, r)$, la concentration réelle du médicament dans le plasma est représentée par $Drug_{real}$, une localisation spatiale est représentée par r, la donnée de distribution du débit de dose de référence est représentée par $Dose\_rate(Drug_{ref}, r)$, la concentration de référence du médicament s dans le plasma est représentée par $Drug_{ref}$, et la variation du débit de dose en fonction de la concentration du médicament dans la donnée de distribution du plasma est représentée par $\frac{\partial Dose\_rate(Drug, r)}{\partial Drug}$.

4.  Le système de contrôle de dose (101) selon la revendication 2, dans lequel le dispositif de traitement est en outre configuré pour obtenir une intensité de faisceau réelle du dispositif d'irradiation (103) ;
dans lequel l'ensemble des coefficients de correction comprend en outre une intensité de faisceau de référence correspondant à la concentration de médicament de référence dans le plasma et à la donnée de distribution du débit de dose de référence.

5.  Le système de contrôle de dose (101) selon la revendication 4, dans lequel le calcul de la donnée de distribution du débit de dose corrigée utilise la formule suivante :

$$Dose\_rate(Beam_{real}, Drug_{real}, r)$$
$$= \frac{Beam_{real}}{Beam_{ref}}\left[Dose\_rate(Beam_{ref}, Drug_{ref}, r)\right.$$
$$\left.+ \frac{\partial Dose\_rate(Beam_{ref}, Drug, r)}{\partial Drug}(Drug_{real} - Drug_{ref})\right]$$

dans lequel la donnée de distribution du débit de dose corrigée est représentée par $Dose\_rate(Beam_{real}, Drug_{real}, r)$, l'intensité réelle du faisceau est représentée par $Beam_{real}$, la concentration réelle du médicament dans le plasma est représentée par $Drug_{ref}$, une localisation spatiale est représentée par r, la donnée de distribution du débit de dose de référence est représentée par $Dose\_rate(Beam_{ref}, Drug_{ref}, r)$, l'intensité du faisceau de référence est représenté par $Beam_{ref}$, la concentration de référence du médicament dans le plasma est représentée par $Drug_{ref}$, et la donnée de distribution de la variation du débit de dose en fonction de la concentration du médicament dans le plasma est représentée par $\frac{\partial Dose\_rate(Beam_{ref}, Drug, r)}{\partial Drug}$.

6.  Le système de contrôle de dose (101) selon la revendication 4, dans lequel l'intensité réelle du faisceau est un flux de neutrons obtenu à l'aide d'un dispositif de mesure de neutrons, ou un courant de protons obtenu à l'aide d'un dispositif de mesure de faisceau (105) configuré par un accélérateur.

7.  Le système de contrôle de dose (101) selon la revendication 2, dans lequel le dispositif de traitement est en outre configuré pour exécuter les étapes suivantes :

    création d'une fonction de concentration du médicament dans le plasma dépendant du temps, basée sur de multiples concentrations réelles du médicament dans le plasma, et création d'une fonction d'intensité du faisceau dépendant du temps, basée sur de multiples intensités réelles du faisceau, dans laquelle les concentrations réelles du médicament dans le plasma et les intensités réelles du faisceau sont obtenues à plusieurs moments au cours de l'irradiation ;

calcul d'une fonction de distribution du débit de dose dépendant du temps basée sur la fonction de concentration du médicament dans le plasma dépendant du temps, la fonction d'intensité du faisceau dépendant du temps et l'ensemble des coefficients de correction ;

calcul d'une fonction dépendant du temps de la distribution de dose accumulée basée sur la fonction dépendant du temps de la distribution du débit de dose ;

du temps d'irradiation restant basé sur la distribution de débit de dose prescrite, la fonction temporelle de la distribution de dose accumulée et la fonction temporelle de la distribution de débit de dose ; et

contrôler le dispositif d'irradiation (103) pour irradier le patient pendant le temps d'irradiation restant.

8. Le système de contrôle de dose (101) selon la revendication 7, dans lequel le calcul de la fonction dépendant du temps de la distribution du débit de dose utilise la formule suivante :

$$Dose\_rate(Beam(t), Drug(t), r)$$

$$= \frac{Beam(t)}{Beam_{ref}} \left[ Dose\_rate(Beam_{ref}, Drug_{ref}, r) \right.$$

$$\left. + \frac{\partial Dose\_rate(Beam_{ref}, Drug, r)}{\partial Drug} (Drug(t) - Drug_{ref}) \right]$$

où la fonction de distribution du débit de dose en fonction du temps est représentée par $Dose\_rate(Beam(t)$, $Drug(t), r)$, la fonction d'intensité du faisceau en fonction du temps est représentée par $Beam(t)$, la fonction de concentration du médicament dans le plasma en fonction du temps est représentée par $Drug(t)$, une position spatiale est représentée par $r$, la donnée de distribution du débit de dose de référence est représentée par $Dose\_rate(Beam_{ref}, Drug_{ref}, r)$, l'intensité du faisceau de référence est représentée par $Beam_{ref}$, la concentration de médicament de référence dans le plasma est représentée par $Drug_{ref}$, la variation du débit de dose en fonction de la concentration du médicament dans la donnée de distribution du plasma est représentée par $\frac{\partial Dose\_rate(Beam_{ref}, Drug, r)}{\partial Drug}$ ; et

dans lequel le calcul de la fonction dépendant du temps de la distribution de dose accumulée utilise la formule suivante :

$$Dose(t, r) = \int Dose\_rate(Beam(t), Drug(t), r) \, dt$$

dans laquelle la fonction dépendante du temps de la distribution de dose accumulée est représentée par $Dose(t, r)$, et la fonction dépendante du temps de la distribution du débit de dose est représentée par $Dose\_rate(Beam(t), Drug(t), r)$.

100

FIG. 1

200

| obtain the real drug concentration in plasma of the patient | —201 |

↓

| calculate the corrected dose rate distribution datum based on the real drug concentration in plasma and a set of correction coefficients | —202 |

↓

| calculate the irradiation time based on the corrected dose rate distribution datum and the prescribed dose distribution datum | —203 |

↓

| cause the irradiation device to irradiate the patient for the irradiation time | —204 |

FIG. 2

EP 4 321 209 B1

<u>300</u>

| create the time-dependent function of drug concentration in plasma based on real drug concentrations in plasma obtained at multiple time points during the irradiation, and create the time-dependent function of beam intensity based on real beam intensities obtained at multiple time points during the irradiation | ~301 |

↓

| calculate the function of dose rate distribution change with time based on the time-dependent function of drug concentration in plasma, the time-dependent function of beam intensity, and the set of correction coefficients | ~302 |

↓

| calculate the time-dependent function of accumulated dose distribution based on the function of dose rate distribution change with time | ~303 |

↓

| calculate the remaining irradiation time based on the prescribed dose rate distribution datum, the time-dependent function of accumulated dose distribution, and the function of dose rate distribution change with time | ~304 |

↓

| cause the irradiation device to irradiate the patient for the remaining irradiation time | ~305 |

FIG. 3

EP 4 321 209 B1

**EP 4 321 209 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022002223 A1